(19) Europäisches Patentamt
European Patent Office
Office européen des brevets

(11) EP 0 755 942 B1

(12) **EUROPEAN PATENT SPECIFICATION**

(45) Date of publication and mention of the grant of the patent:
**04.08.1999 Bulletin 1999/31**

(21) Application number: **95910778.0**

(22) Date of filing: **09.03.1995**

(51) Int. Cl.[6]: **C07K 5/087**, C07K 5/107, A61K 38/06, A61K 38/07

(86) International application number:
**PCT/JP95/00389**

(87) International publication number:
**WO 95/24421 (14.09.1995 Gazette 1995/39)**

(54) **N-AMIDINO DERMORPHIN DERIVATIVE**

N-AMIDINO- DERMORPHIN-DERIVAT

DERIVE TYPE N-AMIDINO DE DERMORPHIN

(84) Designated Contracting States:
**AT BE CH DE DK ES FR GB GR IE IT LI NL PT SE**

(30) Priority: **11.03.1994 JP 4098994**

(43) Date of publication of application:
**29.01.1997 Bulletin 1997/05**

(73) Proprietor:
**DAIICHI PHARMACEUTICAL CO., LTD.**
**Chuo-ku, Tokyo 103 (JP)**

(72) Inventors:
- **SAKURADA, Shinobu**
  **Sendai-shi Miyagi 981-32 (JP)**
- **MURAYAMA, Kimie**
  **Niiza-shi Saitama 352 (JP)**
- **NAKANO, Masaharu**
  **Fuji Chemical Industries, Ltd.**
  **Takaoka-shi Toyama 933 (JP)**
- **HONGO, Kazuya**
  **Fuji Chemical Industries, Ltd.**
  **Takaoka-shi Toyama 933 (JP)**
- **TAKESHIMA, Satoko**
  **Fuji Chemical Industries, Ltd.**
  **Takaoka-shi Toyama 933 (JP)**
- **TAKE, Nobuhiro**
  **Fuji Chemical Industries, Ltd.**
  **Takaoka-shi Toyama 933 (JP)**

(74) Representative:
**Godemeyer, Thomas, Dr.**
**Hauptstrasse 58**
**51491 Overath (DE)**

(56) References cited:
**GB-A- 2 166 139** **JP-A- 61 103 898**

- **J.PHARMACOL.EXP.THER., vol. 266, no. 2, 1993, pages 544-50, XP000650567 PAAKKARI P. ET AL.: "Dermorphin Analog Tyr-D-Arg2-Phe-Sarcosine-induced Opoid Analgesia and Respiratory Stimulation"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7/3.6

## Description

Field of the invention

The present invention relates to peptide derivatives exhibiting pharmaceutical activities such as analgesic activity through actions on opioid receptors and the like.

Background Art

The existence of opioid receptors to which opioids such as morphine bind was verified in the early 1970's. At present, opioid receptors are mainly classified into three types, i.e., μ, δ and κ. Morphine mostly acts on the μ acceptor as an agonist and exhibits pharmaceutical activities such as analgesic activity, enterokinetic inhibition, and respiratory inhibition.

Since 1975, several endogenous morphine-like substances that bind to the opioid receptors have been successively discovered. All of these substances found to date are peptide compounds and are collectively referred to as opioid peptides. The pharmaceutical activities of the opioid peptides are believed to be basically the same as those of morphine. They are potentially safer drugs than morphine since they naturally exist in living bodies. However, natural opioid peptides have problems from the pharmacokinetical standpoint, and they have not been used as clinical medicaments.

In the 1980's, Dermorphine that contains D-alanine residue was isolated from cutises of frogs. It was found that Dermorphine has about 1,000-fold higher analgesic effect than morphine at intraventricular administration and is relatively stable in living bodies. Since then, synthetic opioid peptides containing D-amino acid residues have been prepared. In particular, synthetic opioid peptides with high κ acceptor selectivity are seen as hopeful non-narcotic analgesics and clinical trials have begun. However, the probability of their success as clinical medicaments is doubtful from the viewpoints of efficacy, possible side effects due to properties as κ agonists, and commercial practicability.

Furthermore, it is impossible to use these synthesized opioid peptides as orally available medicaments, and accordingly, they can not be substitutive drugs for MS contin, e.g., which is an orally available controlled release preparation comprising morphine sulfate that has been widely used recently as a medicament for the treatment of cancerous pain. Daily doses of MS contin may occasionally be increased up to gram order, which sometimes leads to difficulty in oral administration. In some cases, its administration cannot be continued because of side effects such as pruritus due to its activity on the release of histamine.

P.Paakkari, et al., The Journal of Pharmacology and Experimental Therapeutics, Vol. 266, No. 2, 1993, p. 544 to p. 550 describes dermorphin analog Tyr-D-$Arg^2$-Phe-Sarcosine-Induced opioid analgesia and respiratory stimulation. The article discloses the compound TAPS having no amidino group on the N-terminal tyrosine residue. It is reported that the peptide has potent opioid effects such as analgesia and is for example about 6 times as potent as dermorphin. TAPS can maintain sufficient analgesic effects for 3 hours by oral administration.

GB 2 166 139 A describes biologically active penta- and heptapeptides which can be used as therapeutic agents. These peptides show interesting pharmacological activities particularly on the central nervous system as analgesics, antipsychotics and neuroendocrinologicals. In the document peptide compounds are described having an amidino group as well as compounds which do not have an amidino group.

Therefore, substitutive medicaments are desired which have higher safety and efficacy than morphine.

Disclosure of the Invention

In order to achieve the aforementioned objects, the inventors of the present invention conducted various studies aimed at providing opioid peptide derivatives having excellent analgesic activity and oral absorbability. As a result, they found that oligopeptide derivatives and their salts having a basic structure of L-Tyr-(L or D)-Arg-Phe and an amidino group at their N-terminals have the desired properties. The present invention was achieved on the basis of these findings.

The peptide derivatives of the present invention can be represented by the following Formula I.

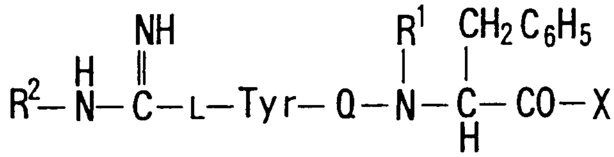

Best Mode for Carrying Out the Invention

The substituents in the above formula will be explained below. Q represents D-Arg (D-arginine residue) or L-Arg (L-arginine residue) and $R^1$ represents hydrogen atom or a $C_{1-6}$ (1 to 6 carbon atoms) alkyl group. Compounds wherein Q is D-Arg and $R^1$ is hydrogen atom are preferred. $R^2$ represents hydrogen atom, a $C_{1-6}$ alkyl group, an aryl group, a $C_{1-6}$ alkanoyl group, or an arylcarbonyl group. For example, aryl groups such as substituted or non-substituted phenyl groups, preferably non-substituted phenyl group; alkanoyl groups such as acetyl group or propanoyl group; and aryl carbonyl groups such as benzoyl group may be used as the aryl group, alkanoyl group, and arylcarbonyl group.

X represents any one of $-OR^3$, $-NR^4R^5$, and $-NR^6-CR^7R^8R^9$. $R^3$ represents hydrogen atom or a $C_{1-6}$ alkyl group, and $R^4$ represents hydrogen atom or a $C_{1-6}$ alkyl group. $R^5$ represents a $C_{1-6}$ hydroxyalkyl group or a sulfonic acid-substituted $C_{1-6}$ alkyl group. Although the hydroxyl group or sulfonic acid group may substitute on any position of the alkyl group, terminally substituted alkyl groups are preferred. $R^4$ and $R^5$ may combine together with the nitrogen atom to which $R^4$ and $R^5$ bind to form a 5 or 6-membered nitrogen-containing saturated heterocyclic group. The heterocyclic group may contain two or more nitrogen atoms. For example, 1-piperazinyl group, 1-pyrrolidinyl group, or 1-piperidinyl group may be used as $-NR^4R^5$.

Where X is $-NR^6-CR^7R^8R^9$, $R^6$ represents hydrogen atom, a $C_{1-6}$ alkyl group, or a $C_{1-6}$ alkyl group substituted with an aryl group such as phenyl group (aralkyl group). An example of the aralkyl group is benzyl group. $R^7$ represents hydrogen atom; carboxyl group; a carbonyl group substituted with a $C_{1-6}$ alkoxy group such as methoxy carbonyl or ethoxy carbonyl group; a substituted or non-substituted carbamoyl group; a $C_{1-6}$ alkyl group substituted with a carboxyl group; a $C_{1-6}$ alkyl group substituted with a substituted or non-substituted carbamoyl group; or a $C_{1-6}$ alkyl group having a carbonyl group substituted with a $C_{1-6}$ alkoxy group.

$R^8$ represents hydrogen atom; a $C_{1-6}$ alkyl group; an amino $C_{1-6}$ alkyl group; a $C_{1-6}$ alkyl group substituted with an amidino group; a $C_{1-6}$ alkyl group substituted with a guanidino group; a hydroxy-$C_{1-6}$ alkyl group, a carboxy-substituted $C_{1-6}$ alkyl group, or a $C_{1-6}$ alkyl group substituted with a substituted or non-substituted carbamoyl group. Alternatively, $R^6$ and $R^8$ may combine to form, together with the nitrogen atom to which $R^6$ binds, a 5 or 6-membered nitrogen-containing saturated heterocyclic group having a carboxyl group on its ring. Examples of the heterocyclic group include 2-carboxy-1-pyrrolidinyl group (-Pro-OH) and 3-carboxy-1-piperidinyl group. Combinations where $R^7$ is carboxyethyl group or carbamoylethyl group and $R^8$ is hydrogen atom are preferred. $R^9$ represents hydrogen atom or $C_{1-6}$ alkyl group. In the aforementioned substituents, the alkyl group, alkoxy group, or alkanoyl group may be either straight or branched.

The compounds of the present invention represented by Formula I have an asymmetric carbon atom derived from phenylalanine residue attached to Q, an asymmetric carbon atom substituted with $R^7$ and $R^8$ (except when $R^7$ and $R^8$ simultaneously represent the same substituents), and one or more asymmetric carbon atoms which may optionally exist in each of the above-described substituents, as well as two asymmetric carbon atoms derived from L-tyrosine residue and D-Arg or L-Arg residue represented by symbol Q. The asymmetric carbon atoms other than those derived from residues of L-Tyr, D-Arg, and L-Arg may have either R- or S-configuration. Any optical isomers or racemates, diastereomers, and mixtures of such isomers fall within the scope of the compounds of the present invention according to Fomula I.

Acid addition salts such as hydrochlorides, acetates, or para-toluenesulfonates, or base addition salts such as ammonium salts or organic amino salts also fall within the compounds of the present invention. In addition to the compound represented by the above formula, dimers or oligomers of the above-described compounds and cyclic compounds in which C-termial and N-terminal of these compounds bind also fall within the compounds of the present invention.

The peptides of the present invention have higher analgesic activity than morphine. Since their analgesic activity is accompanied by weaker effects on the release of histamine and heart rate depression than those of morphine, and since their degree of cross resistance with morphine is low, they can be expected to be suitable for the treatment of cancerous pain. Examples of the route of administration include, for example, intravenous administration, subcutaneous administration, and oral administration. Formulations for mucosal absorption including nasal absorption and formulations for endermic absorption are also expected to be useful.

The peptide derivatives of the present invention can be prepared by solid phase methods and liquid phase methods ordinarily used for peptide preparations. For example, peptide chains may first be prepared without amidino groups by the solid phase method, and then amidino groups may be introduced to the amino group of N-terminal tyrosine to obtain desired peptide derivatives. Alternatively, amidino groups may be introduced beforehand and the C-terminal then be modified. Various excellent agents are available as protective groups for amino groups and the like and as condensing agents or the like for condensation reactions. These can be suitably selected with reference to Examples set out below, or in view of, for example, Koichi Suzuki Ed., "Tanpakushitu Kohgaku-Kiso To Ohyo (Protein Engineering: Fundamentals and Applications)", Maruzen Co., Ltd. (1992) and publications cited therein; M. Bodanszky, et al., "Peptide Synthesis", John Wiley & Sons, N.Y., 1976; and J.M. Stewart and D.J. Young, "Solid Phase Peptide Synthesis", W.H. Freeman and Co., San Francisco, 1969. For the solid phase methods, various commercially available peptide synthesizers, for example, Model 430A (Perkin Elmer Japan, formerly Applied Biosystems), may conveniently be used. Resins, reagents and the like used in the preparations are easily obtainable as commercially available products and examples are indicated in Examples.

## Examples

The present invention will be further explained by way of examples. However, the present invention is not limited to these examples. By referring to the examples, modifying or altering the methods of the present examples, or appropriately selecting starting materials or reagents for reactions, desired peptide derivatives of the present invention that fall within the scope of Formula I can easily be prepared. In the examples, the meanings of amino acid groups are similar to those ordinarily used. Where an amino acid having D-form and L-form is referred to, the amino acid represents an L-amino acid unless specifically stated to be D-form. In addition, the following abbreviations will be used, and similar abbreviations not specifically defined here will occasionally be used.

| | |
|---|---|
| Z | : benzyloxycarbonyl group |
| OTce | : trichloroethyl ester group |
| Boc | : t-butoxycarbonyl group |
| WSCI | : 1-ethyl-3-(3-dimethylaminopropyl)-carbodiimide |
| TsoOH | : para-toluenesulfonic acid |
| OBzl | : benzyloxy group |
| Me β Ala or β MeAla | : N-methyl-β-alanine |
| H-β Ala-ol | : $NH_2CH_2CH_2CH_2OH$ |
| Fmoc | : 9-fluorenylmethyloxycarbonyl |
| Pmc | : 2,2,5,7,8-pentamethylchroman-6-sulfonyl |
| t-Bu | : tertiary-butyl |
| NMP | : N-methylpyrrolidone |
| DMF | : dimethylformamide |
| DMSO | : dimethylsulfoxide |
| TFA | : trifluoroacetic acid |
| TEA | : triethylamine |
| DCM | : dichloromethane |
| DMAP | : N,N-dimethylaminopyridine |
| DIPEA | : N,N-diisopropylethylamine |
| DIPCI | : N,N-diisopropylcarbodiimide |
| HOBt | : 1-hydroxybenzotriazole |
| EDC | : 1-(3-dimethylaminopropyl)-3-ethyl-carbodiimide |
| HBTU | : 2-(1H-benzotriazole-1-yl)-1,1,3,3-tetramethyluronium hexafluorophosphate |
| PyBrop | : bromotris(pyrrolidino)phosphonium hexafluorophosphate |
| Alko resin | : p-alkoxybenzylalcohol resin (4-hydroxymethylphenoxymethyl-co-polystyrene 1% divinylbenzene resin, J. Am. Chem. Soc., 95, 1328 (1974), Watanabe Chemical Industry) |
| Fmoc-NH-SAL resin | : 4-(2',4'-dimethoxyphenyl-9-fluorenylmethoxycarbonylamionomethyl)phenoxy resin (Watanabe Chemical Industry) |

Example 1

$H_2NC(NH)$-Tyr-D-Arg-Phe-$NHCH_2CH_2COOH$

The above peptide was prepared by the solid phase method (Original Autoprogram for the Fmoc/NMP method) using a Model 430A peptide synthesizer manufactured by Applied Biosystems Inc. (ABI) as follows.

Fmoc-β-Ala-Alko resin (0.25 mmol/675 mg) was washed once with NMP, and treated with NMP containing 20% piperidine for 4 minutes, and then with NMP containing 20% piperidine in the same manner for 16 minutes. The resin was washed with NMP 5 times, and then allowed to react with Fmoc-Phe-OH for 61 minutes. After being washed with NMP 4 times, the resin was recovered from the fourth rinse, and unreacted amino groups were allowed to react with acetic anhydride.

The above-described first cycle was carried out in 120 minutes, and a similar procedure was conducted using Fmoc-D-Arg(Pmc)-OH for Fmoc-Phe-OH in the second cycle, and Fmoc-Tyr(t-Bu)-OH in the third cycle. As side chain protective groups, Pmc was used for D-Arg and t-Bu for Tyr.

500 mg of the resin obtained in the above procedure was treated with stirring in a mixture of phenol (crystal, 0.75 g), ethanedithiol (0.25 ml), thioanisole (0.50 ml), water (0.50 ml) and TFA (10.0 ml) for 3 hours at room temperature to release peptides from the resin and simultaneously remove the protective groups. Then, the mixture was filtered using a 3 μm filter (ADVANTEC-Polyflon filter), cold diethyl ether (200 ml) was added to the filtrate, and the resulting precipitates were collected by filtration using a 3 μm filter (ADVANTEC-Polyflon filter). The precipitates on the filter were dissolved in 10-20 ml of 2N acetic acid and then lyophilized to give crude peptide.

The crude peptide (135 mg) was dissolved in water (13.5 ml), and 1M o-methylisourea (13.5 ml) was added. Stirring was continued for 14 days at 4 °C for amidination. The resulting crude amidinated peptide (106 mg) was dissolved in 20 ml of 0.1% aqueous solution of TFA. After adjustment of pH to 4-5 with 0.01N hydrochloric acid, the peptide was purified using a Gilson HPLC system. For the HPLC, a Cosmosil $C_{18}$ column was used, and a continuous linear gradient consisting of a mixture of 0.1% aqueous TFA solution and 0.1% aqueous TFA solution containing 70% acetonitrile (mixing ratio was from 0% at time zero to 60% at 45 minutes) was used at the flow rate of 10 ml/minute.

About 100 μg of the purified peptide was applied to a hydrolysis tube and 6N hydrochloric acid containing 0.2% phenol (1 ml) was added. After evacuation and sealing of the tube, hydrolysis was carried out for 24 hours at 110°C. After cooling to ambient temperature and evaporation to dryness at 40°C, the result was dissolved in 0.01N hydrochloric acid (1 ml) and 20 μl of the solution was subjected to amino acid analysis using an amino acid analyzer [analyzer: Hitachi L-8500, Column: Hitachi Custom Ion Exchange Resin # 2622, pre-column: Hitachi Custom Ion Exchange Resin # 2650L, buffer: lithium buffer (0.09N-pH 2.8, 0.25N-pH3.7, 0.72N-pH 3.6, 1.00N-pH 4.1, regeneration 0.20N), reaction temperature: 135 °C, flow rate of buffer pump: 0.30 ml/minute, flow rate of ninhydrin pump: 0.35 ml/minute, wavelength of detector: 570 nm/440 nm, assay time: 150 minutes]. The result of the amino acid analysis verified the above-mentioned structure.

The purified peptide (about 150 μg) was dissolved in 5% acetic acid (250 μl) and 1 μl of the solution was subjected to mass spectrometric analysis using Liquid SIMS [MS and MS/MS, cesium ion gun was used, analyzer: Finnigan TSQ 700, matrix: glycerol-thioglycerol (1:1), collision gas: Ar gas (3-5 mTorr), collision energy: -20 eV, electron multiplier: 1000 to 1500 V]. The results obtained verified the above-described structure.

Example 2

$H_2NC(NH)$-Tyr-D-Arg-Phe-$NHCH_2CH_2CONH_2$

The above peptide derivative was obtained in the same manner as in Example 1, except that Fmoc-NH-SAL resin (0.25 mmol/385 mg) was used at the beginning of the synthesis, Fmoc-β-Ala-OH in the first cycle, Fmoc-Phe-OH in the second cycle, Fmoc-D-Arg(Pmc)-OH in the third cycle, and Fmoc-Tyr(t-Bu)-OH in the fourth cycle.

Synthesis and purification of the amidinated peptide derivative were also carried out in the same manner as in Example 1 except for use of a mixture of 0.05% aqueous formic acid solution and 0.05% aqueous formic acid solution containing 70% acetonitrile (a continuous linear gradient with a mixing ratio of from 0% at the beginning of HPLC to 40% at 45 minutes). Amino acid analysis and mass spectrometric analysis were also carried out in the same manner as in Example 1, and the results verified the aforementioned structure.

Example 3

$H_2NC(NH)$-Tyr-D-Arg-Phe-$N(CH_3)CH_2CH_2COOH$

The tetrapeptide derivative having the above structure was prepared by the solid phase method according to

the Fmoc/NMP method as follows. A glass filter was used for filtration.

After Alko resin (0.500 g) had been swollen with DMF (6 ml), the resin was added with Fmoc-N-methyl-β-alanine (Fmoc-β-MeAla-OH, 0.228 g) and pyridine (0.093 ml), shaken for 1 minute, and then added with 2,6-dichlorobenzoyl chloride (0.147 g) and shaken for 24 hours. The resulting Fmoc-β-MeAla-Alko resin was washed 3 times with 6 ml of DMF, then 3 times with 6 ml of methanol and further 3 times with 6 ml of DCM, and unreacted hydroxymethyl groups were benzoylated by adding benzoyl chloride (0.0891 ml) and pyridine (0.0847 ml) in DCM (6 ml) and shaking for 1 hour. The amino acid resin was successively washed 3 times with 6 ml of DCM, 3 times with 6 ml of DMF and 3 times with 6 ml of methanol and vacuum dried in a desiccator over potassium hydroxide.

The Fmoc-β-MeAla-Alko resin was treated 3 times with 12 ml of DMF, then 3 times with 12 ml of DMF containing 20 % piperidine and further 6 times with 12 ml of DMF to remove the Fmoc group, and then added with Fmoc-Phe-OH (0.262 g), PyBrop (Watanabe Chemical Industry, 0.315 g), NMP (6 ml) and DIPEA (0.273 ml) and shaken for 24 hours to form Fmoc-Phe-β-MeAla-Alko resin. After filtration and washing with NMP (6 ml), unreacted amino groups were capped by treatment with DMF (6 ml) containing 1-acetylimidazole (0.248 g) and DIPEA (0.0784 ml) for 1 hour. The resulting resin was then washed with NMP (6 ml).

The Fmoc group was removed from the Fmoc-Phe-β-MeAla-Alko resin in the same manner as described above, and the result was added with Fmoc-D-Arg(Pmc)-OH (0.557 g), HOBt (0.121 g), HBTU (0.299 g) and DIPEA (0.274 ml) and shaken for 1 hour to form Fmoc-D-Arg(Pmc)-Phe-β-MeAla-Alko resin. Subsequently, after filtration and washing, unreacted amino groups were capped in the same manner as described above.

The Fmoc group was removed from the Fmoc-D-Arg(Pmc)-Phe-β-MeAla-Alko resin obtained in the same manner as described above, and the result was added with Fmoc-Tyr(t-Bu)-OH (0.310 g), HOBt (0.103 g), HBTU (0.256 g) and DIPEA (0.235 ml) and shaken for 1 hour to form Fmoc-Tyr(t-Bu)-D-Arg(Pmc)-Phe-β-MeAla-Alko resin. After filtration and washing in the same manner as described above, unreacted amino groups were capped.

The Fmoc group was removed from the Fmoc-Tyr(t-Bu)-D-Arg(Pmc)-Phe-β-MeAla-Alko resin in the same manner as described above, and the result was added with 1H-pyrazole-1-carboxyamidine hydrochloride (0.989 g) prepared according to the method of Matsueda et al. (The Journal of Organic Chemistry, 57, 2497-2502, 1992), DIPEA (1.293 ml) and DMF (6 ml), and allowed to react at from 10 to 60 °C, more preferably, at from 40 to 50 °C, for from 1 to 4 hours to amidinate the amino group of the N-terminal tyrosine. Then, the result was filtered, washed (3 times with 6 ml of NMP and further 3 times with 6 ml of methanol) and vacuum dried in a desiccator over potassium hydroxide.

The resin (596 mg) obtained by the above procedure was placed on a glass filter and treated with a mixture of TFA, phenol and water (5 ml, TFA:phenol:water = 93:2:5) for 1 hour and filtered. The same treatment was repeated twice. The resin was further treated with TFA (5 ml) for 5 minutes and filtered. The same treatment was repeated 3 times. The filtrates obtained by the treatments were combined and the solvent was removed under reduced pressure at a temperature below 20°C. After repeating a treatment in which residue was added with diethyl ether (20 ml) to form white precipitates and the supernatant was discarded, the resulting white powder was dissolved in water (20 ml), washed with diethyl ether (5 ml) 3 times in a separation funnel, and the aqueous layer was lyophilized to obtain crude amidino peptide.

The crude amidino peptide (56.2 mg) was dissolved in a 0.05% aqueous TFA solution (10 ml) and purified by a Shimazu HPLC system. For the HPLC, a YMC D-ODS-5-ST $C_{18}$ column and a mixture of 0.05% aqueous TFA solution containing 0.5% acetonitrile and 0.05% aqueous TFA solution containing 70% acetonitrile (a linear gradient at a mixing ratio of from 0% at the beginning of HPLC to 90% at 50 minutes at the flow rate of 1 ml/minute) were used. Amino acid analysis and mass spectrometric analysis were carried out in the same manner as in Example 1. The results verified the above structure.

Example 4

$H_2NC(NH)$-Tyr-D-Arg-Phe-$N(CH_2CH_3)CH_2CH_2COOH$

The tetrapeptide derivative having the above structure was prepared by the solid phase method according to the Fmoc/NMP method as follows. A glass filter was used for filtration.

After Alko resin (1.000 g) had been swollen with DMF (12 ml), the resin was added with Fmoc-N-ethyl-β-alanine (Fmoc-β-EtAla-OH, 0.475 g) and DMAP (0.017 g), shaken for 1 minute, then added with DIPCI (0.177 g) and shaken for 24 hours. The resulting Fmoc-β-EtAla-Alko resin was washed 3 times with 12 ml of NMP, then 3 times with 12 ml of methanol and further 3 times with 12 ml of DCM, and unreacted hydroxymethyl groups were benzoylated by adding benzoyl chloride (0.178 ml) and pyridine (0.170 ml) in DCM (12 ml) and shaking for 1 hour. Then, the amino acid resin was successively washed 3 times with 12 ml of DCM, 3 times with 12 ml of DMF, and 3 times with 12 ml of methanol and vacuum dried in a desiccator over potassium hydroxide.

The Fmoc-β-EtAla-Alko resin was treated 3 times with 20 ml of DMF, then 3 times with 12 ml of DMF containing 20 % piperidine and further 6 times with 12 ml of DMF to remove the Fmoc groups, and added with Fmoc-Phe-OH

(0.387 g), PyBrop (0.466 g), NMP (12 ml) and DIPEA (0.523 ml) and shaken for 24 hours to afford Fmoc-Phe-β-EtAla-Alko resin. After filtration, the result was washed with NMP (12 ml) and unreacted amino groups were capped by treatment with DMF (12 ml) containing 1-acetylimidazole (0.551 g) and DIPEA (0.174 ml) for 1 hour. Then, the resulting resin was again washed with NMP (12 ml).

The Fmoc group was removed from the Fmoc-Phe-β-EtAla-Alko resin obtained above in the same manner as described above. The resin was added with Fmoc-D-Arg(Pmc)-OH (0.707 g), HOBt (0.153 g), HBTU (0.379 g) and DIPEA (0.348 ml) and shaken for 1 hour to obtain Fmoc-D-Arg(Pmc)-Phe-β-EtAla-Alko resin. Unreacted amino groups were capped after filtration and washing in the same manner as described above.

The Fmoc group was removed from the Fmoc-D-Arg(Pmc)-Phe-β-EtAla-Alko resin in the same manner as described above. The resin was added with Fmoc-Tyr(t-Bu)-OH (0.460 g), HOBt (0.153 g), HBTU (0.399 g) and DIPEA (0.348 ml) and shaken for 1 hour to form Fmoc-Tyr(t-Bu)-D-Arg(Pmc)-Phe-β-EtAla-Alko resin. After filtration and washing, unreacted amino groups were capped in the same manner as described above.

The Fmoc group was removed from the Fmoc-Tyr(t-Bu)-D-Arg(Pmc)-Phe-β-EtAla-Alko resin in the same manner as described above. The resin was added with 1H-pyrazole-1-carboxyamidine hydrochloride (2.199 g) prepared in the same manner as in Example 3, DIPEA (2.874 ml) and DMF (6 ml) and allowed to react at 40 to 50 °C for 1 to 4 hours for the amidination of amino groups at N-terminal tyrosine. Then, the result was filtered, washed (3 times with 12 ml of NMP and further 3 times with 12 ml of methanol) and vacuum dried in a desiccator over potassium hydroxide.

The resin (1.514 g) obtained by the procedure described above was placed on a glass filter and treated with 10 ml of a mixture containing TFA, phenol, and water (TFA:phenol:water = 93:2:5) for 1 hour and filtered. The same treatment was repeated twice. The resin was further treated with TFA (10 ml) for 5 minutes and filtered. The same treatment was repeated 3 times. The filtrates obtained from the treatments were combined and the solvent was removed under reduced pressure at a temperature below 20°C. A treatment in which the residue was added with diethyl ether (20 ml) to form white precipitates and the supernatant was discarded was repeated 3 times. The resulting white powder was dissolved in water (30 ml), washed with 5 ml of diethyl ether 3 times in a separation funnel and the aqueous layer was lyophilized to obtain crude amidino peptide.

The crude amidino peptide (100 mg) was dissolved in a 0.05% aqueous TFA solution (20 ml) and purified in the same manner as in Example 3. Amino acid analysis and mass spectrometric analysis were carried out in the same manner as in Example 1. The results verified the above-described structure.

Example 5

$H_2$NC(NH)-Tyr-D-Arg-Phe-OMe
$H_2$NC(NH)-Tyr-D-Arg-Phe-NHMe
$H_2$NC(NH)-Tyr-D-Arg-Phe-β Ala-ol
$H_2$NC(NH)-Tyr-D-Arg-Phe-Gly-OH
$H_2$NC(NH)-Tyr-D-Arg-Phe-Ala-OH
$H_2$NC(NH)-Tyr-D-Arg-Phe-Me β Ala-OEt
$H_2$NC(NH)-Tyr-D-Arg-Phe-(n-Pr) β Ala-OH

As a starting material for the preparation of the above peptides, the protected peptide represented by the formula Z-HNC(N-Z)-Tyr(Bzl)-D-Arg($Z_2$)-Phe-OH was prepared by a liquid method as follows:

The starting material, Z-Phe-OTce (254 g), was treated with 25% hydrogen bromide-acetic acid (900 ml) to remove Z group and then dissolved in $CH_2Cl_2$ (1000 ml) on an ice bath. After this solution was added with Boc-D-Arg($Z_2$)-OH (288 g) and HOBt (85 g) and neutralized with TEA (77 ml), condensation reaction was carried out by using EDC • HCl (121 g) to form Boc-D-Arg($Z_2$)-Phe-OTce. Then, Boc-D-Arg($Z_2$)-Phe-OTce (241 g) was treated with 4N HCl-ethyl acetate (1000 ml) to remove Boc group and dissolved in DMF (1300 ml) on an ice bath. After the solution was added with Boc-Tyr(Bzl)-OH (108 g) and HOBt (46 g) and neutralized with TEA (42 ml), condensation reaction was carried out using EDC • HCl (65 g) to obtain a protected peptide of the formula Boc-Tyr(Bzl)-D-Arg($Z_2$)-Phe-OTce.

The Boc-Tyr(Bzl)-D-Arg($Z_2$)-Phe-OTce (48 g) was treated with 4N HCl-ethyl acetate (250 ml) to remove Boc group and then dissolved in DMF (150 ml) on an ice bath. The solution was neutralized with TEA (7 ml) and added with Z-HNC(N-Z)-pyrazole (amidination reagent, 19 g) and stirred at room temperature to obtain the protected peptide Z-HNC(N-Z)-Tyr(Bzl)-D-Arg($Z_2$)-Phe-OTce. This protected peptide (42 g) was dissolved in acetic acid on a water bath, added with zinc powder (21 g, 0.32 ml) and stirred for 2 hours. After the zinc powder was removed from the reaction mixture, concentration under reduced pressure gave the protected peptide Z-HNC(N-Z)-Tyr(Bzl)-D-Arg($Z_2$)-Phe-OH.

The Z-HNC(N-Z)-Tyr(Bzl)-D-Arg($Z_2$)-Phe-OH was condensed with MeOH (EDC-DMAP method); $NH_2Me$ (EDC-HOBt method); H-β Ala-ol(EDC-HOBt method); H-Gly-OBzl • TosOH (EDC-HOBt method); H-Ala-OBzl • TosOH (EDC-HOBt); H-Me β Ala-OEt (EDC-HOBt method); or H-(n-Pr) β Ala-OBzl • TosOH (EDC-HOBt method). Each of the resulting products was dissolved in acetic acid on a water bath and subjected to catalytic hydrogenation in the presence

of palladium on carbon. The catalyst was removed and the reaction mixture was concentrated under reduced pressure and subsequently lyophilized to give the desired peptides in the form of powder. The results of amino acid analysis and mass spectrometric analysis under the conditions set out below verified the structure of each resulting peptides.

For the amino acid analysis, the peptide (approximately 0.5 mg) was placed in a hydrolysis tube and added with 6N hydrochloric acid (1 ml). After evacuation and sealing of the tube, hydrolysis was carried out for 24 hours at 110 °C. After cooling to ambient temperature, the result was concentrated to dryness at 40 °C, and then the residue was dissolved in purified water (5 ml). A portion (5μl) was sampled from the solution and dried under reduced pressure, and then the residue was added with 20 μ l of 50 mM $NaHCO_3$ buffer (pH 9.0) and 40 μ l of dansyl chloride-acetonitrile solution and warmed at 70 °C for 10 minutes. The reaction mixture was concentrated under reduced pressure and the residue was dissolved in 50% ethanol (100 μ l). The resulting solution (20 μ l) was analyzed by liquid chromatography. For the mass spectrometric analysis, about 150 μ g of the peptide was dissolved in 5% acetic acid (250 μ l) and a portion (1 μ l) was analyzed by Liquid SIMS.

Example 6

$H_2NC(NH)$-Tyr-D-Arg-MePhe-Me β Ala-OH

$H_2NC(NH)$-Tyr-D-Arg-EtPhe-Me β Ala-OH

The above peptides were prepared successively from the C-terminals by a liquid method using TosOH • Me β Ala-OBzl as a starting material. Boc-MePhe-OH and TosOH • Me β Ala-OBzl were condensed by the EDC-HOBt method to afford Boc-MePhe-Me β Ala-OBzl. Boc group was removed from the Boc-MePhe-Me β Ala-OBzl using 4N HCl-ethyl acetate, and the result was condensed with Boc-D-Arg($Z_2$)-OH by the EDC-HOBt method to form Boc-D-Arg($Z_2$)-MePhe-Me β Ala-OBzl. Subsequently, Boc group was removed from the Boc-D-Arg($Z_2$)-MePhe-Me β Ala-OBzl using 4N HCl-ethyl acetate, and the result was condensed with Boc-Tyr(Bzl)-OH by EDC-HOBt method to obtain the protected peptide Boc-Tyr(Bzl)-D-Arg($Z_2$)-MePhe-Me β Ala-OBzl.

The Boc-Tyr(Bzl)-D-Arg($Z_2$)-MePhe-Me β Ala-OBzl was treated with 4N HCl-ethyl acetate to remove Boc group, and then the protected peptide Z-HNC(N-Z)-Tyr(Bzl)-D-Arg($Z_2$)-MePhe-Me β Ala-OBzl was obtained by adding Z-HNC(N-Z)-pyrazole (amidination reagent) and stirring at room temperature. This protected peptide was dissolved in acetic acid. After addition of palladium on carbon, catalytic hydrogenation was carried out by bubbling hydrogen gas into the solution. After the palladium on carbon was removed, concentration under reduced pressure and lyophilization gave the peptide $H_2NC(NH)$-Tyr-D-Arg-MePhe-Me β Ala-OH in the form of powder. By the amino acid analysis and mass spectrometric analysis described in Example 5, the peptide was verified to have the above structure.

By using Boc-EtPhe-OH instead of Boc-MePhe-OH, Boc-EtPhe-Me β Ala-OBzl was prepared by condensation with TosOH • Me β Ala-OBzl by the EDC-HOBt method in a similar manner to those described above. Then, deprotection and condensation were repeated in the same manner as described above to give the protected peptide Z-HNC(N-Z)-Tyr(Bzl)-D-Arg($Z_2$)-EtPhe-Me β Ala-OBzl. This protected peptide was dissolved in acetic acid and added with palladium on carbon. Catalytic hydrogenation was carried out by bubbling hydrogen gas into the solution. After the palladium on carbon was removed, concentration under reduced pressure and lyophilization gave the peptide $H_2NC(NH)$-Tyr-D-Arg-EtPhe-Me β Ala-OH in the form of powder. By the amino acid analysis and mass spectrometric analysis described in Example 5, the peptide was verified to have the above structure.

Example 7

$H_2NC(NH)$-Tyr-D-Arg-Phe-Me β Ala-OH

Preparation was carried out according to a liquid phase method ordinary used for peptide synthesis. Z-Phe-OTce (254 g), used as a starting material, was treated with 25% hydrogen bromide-acetic acid (900 ml) to remove Z group and dissolved in $CH_2Cl_2$ (1000 ml) on an ice bath. This solution was added with Boc-D-Arg($Z_2$)-OH (288 g) and HOBt (85 g) and neutralized with TEA (77 ml), and then condensed with EDC • HCl (121 g) to obtain Boc-D-Arg($Z_2$)-Phe-OTce. Subsequently, the Boc-D-Arg($Z_2$)-Phe-OTce (241 g) was treated with 4N HCl-ethyl acetate (1000 ml) to remove Boc group and then dissolved in DMF (1300 ml) on an ice bath. The solution was added with Boc-Tyr(Bzl)-OH (108 g) and HOBt (46 g) and neutralized with TEA (42 ml), and then condensation was carried out using EDC • HCl (65 g) to obtain a protected peptide represented by the formula Boc-Tyr(Bzl)-D-Arg($Z_2$)-Phe-OTce.

The Boc-Tyr(Bzl)-D-Arg($Z_2$)-Phe-OTce (48 g) was treated with 4N HCl-ethyl acetate (250 ml) to remove Boc group and dissolved in DMF (150 ml) on an ice bath. The solution was neutralized with TEA (7 ml) and added with Z-HNC(N-Z)-pyrazole (amidination reagent, 19 g) and stirred at room temperature to give the protected peptide of the formula Z-HNC(N-Z)-Tyr(Bzl)-D-Arg($Z_2$)-Phe-OTce. This protected peptide (42 g) was dissolved in acetic acid on a water

bath, added with zinc powder (21 g), and then stirred for 2 hours. After the zinc powder was removed from the reaction mixture, concentration under reduced pressure gave the protected peptide Z-HNC(N-Z)-Tyr(Bzl)-D-Arg($Z_2$)-Phe-OH.

This protected peptide Z-HNC(N-Z)-Tyr(Bzl)-D-Arg($Z_2$)-Phe-OH (1.15 g) and TosOH • Me β Ala-OBzl (0.93 g) were dissolved in 30 ml of DMF (containing 10% DMSO) on an ice bath. This solution was added with HOBt (0.24 g) and neutralized with TEA (0.21 ml), and then condensation was carried out using EDC • HCl (0.25 g) to obtain the protected peptide represented by the formula Z-HNC(N-Z)-Tyr(Bzl)-D-Arg($Z_2$)-Phe-Me β Ala-OBzl. The protected peptide (0.90 g) was dissolved in acetic acid (100 ml) and added with palladium on carbon. Catalytic hydrogenation was carried out by bubbling hydrogen gas into the solution. After palladium on carbon was removed, concentration under reduced pressure and lyophilization gave the peptide $H_2$NC(NH)-Tyr-D-Arg-Phe-Me β Ala-OH in the form of powder. By the amino acid analysis and mass spectrometric analysis described in Example 5, the peptide was verified to have the above structure.

As physicochemical properties of the peptide derivatives of the present invention, HPLC retention times and Rf values of thin layer chromatography are shown in Table 1. All of these peptide derivatives were obtained as lyophilized products.

Conditions for HPLC:

| | |
|---|---|
| Apparatus | : Nihon-Bunkou 880 System |
| Column | : Nucleosil 5C18 100 Å |
| Solvent | : Solution A (acetonitrile:purified water:trifluoro acetic acid=10:90:0.1)<br>Solution B (acetonitrile:purified water:trifluoro acetic acid=70:30:0.1) |
| Gradient | : Solution B 0-100%/30 min. |
| Flow Rate | : 1 ml/min. |
| Temperature | : 40°C |
| Wavelength for detection | : 230 nm |
| Injection Volume | : 20 μl of sample (conc. 1 mg/ml) was injected by an automatic sample injector |

Conditions for thin layer chromatography:

| | |
|---|---|
| $Rf^a$ | : n-butanol:acetic acid purified water=4:1:5 was mixed and then the supernatant was used as developing solution. |
| $Rf^b$ | : n-butanol:acetic acid:purified water:pyridine=15:3: 10:12 was used as developing solution. |
| Thin layer plate | : Silica gel (Merck F254) |

Other compounds obtained in the same manner as in the aforementioned examples are shown in Table 2.

Table 1

| Peptide derivatives of the present invention | HPLC (retention time) | $Rf^a$ | $Rf^b$ |
|---|---|---|---|
| $H_2NC(NH)$-Tyr-D-Arg-Phe-$NH(CH_2)_2COOMe$ | 10.79 | 0.49 | 0.74 |
| $H_2NC(NH)$-Tyr-D-Arg-Phe-$N(CH_3)(CH_2)_2COOMe$ | 12.22 | 0.47 | 0.74 |
| $H_2NC(NH)$-Tyr-D-Arg-Phe-OMe | 11.37 | 0.60 | 0.84 |
| $H_2NC(NH)$-Tyr-D-Arg-Phe-$NHCH(CH_3)CH_2OH$ | 9.11 | 0.46 | 0.70 |
| $H_2NC(NH)$-Tyr-D-Arg-Phe-Arg-$NH_2$ | 7.83 | 0.23 | 0.55 |
| $H_2NC(NH)$-Tyr-D-Arg-Phe-Leu-OH | 13.19 | 0.41 | 0.64 |
| $H_2NC(NH)$-Tyr-D-Arg-Phe-OH | 9.35 | 0.50 | 0.76 |
| $H_2NC(NH)$-Tyr-D-Arg-Phe-Gly-OH | 8.54 | 0.37 | 0.60 |
| $H_2NC(NH)$-Tyr-D-Arg-Phe-Asp-OH | 8.41 | 0.25 | 0.44 |
| $H_2NC(NH)$-Tyr-D-Arg-Phe-Ala-OH | 9.27 | 0.43 | 0.66 |

Table 1 (continued)

| Peptide derivatives of the present invention | HPLC (retention time) | Rf[a] | Rf[b] |
|---|---|---|---|
| $H_2NC(NH)$-Tyr-D-Arg-Phe-$N(CH_3)CH_2CH_2OH$ | 9.64 | 0.51 | 0.74 |
| $H_2NC(NH)$-Tyr-D-Arg-Phe-$N(CH_3)CH_2COOMe$ | 11.49 | 0.49 | 0.74 |
| $H_2NC(NH)$-Tyr-D-Arg-Phe-Gly-$NH_2$ | 8.09 | 0.38 | 0.60 |
| $H_2NC(NH)$-Tyr-D-Arg-Phe-$N(CH_3)(CH_2)_2CON(Me)_2$ | 11.11 | 0.35 | 0.69 |
| $H_2NC(NH)$-Tyr-D-Arg-Phe-Pro-OH | 10.68 | 0.33 | 0.54 |
| $H_2NC(NH)$-Tyr-D-Arg-Phe-NHMe | 9.01 | 0.43 | 0.68 |
| $H_2NC(NH)$-Tyr-D-Arg-Phe-$N(CH_3)(CH_2)_2CONH$(n-Hex) | 17.26 | 0.52 | 0.79 |
| $H_2NC(NH)$-Tyr-D-Arg-Phe-NHEt | 10.24 | 0.59 | 0.81 |
| $H_2NC(NH)$-Tyr-D-Arg-Phe-D-Ala-$NH_2$ | 9.09 | 0.40 | 0.67 |
| $H_2NC(NH)$-Tyr-D-Arg-Phe-$N(CH_3)CH(CH_3)COOH$ | 10.81 | 0.42 | 0.67 |
| $H_2NC(NH)$-Tyr-D-Arg-D-Phe-$N(CH_3)CH(CH_3)COOH$ | 11.58 | 0.41 | 0.58 |
| $H_2NC(NH)$-Tyr-D-Arg-Phe-$NHC(CH_3)_2COOH$ | 10.57 | 0.41 | 0.61 |
| $H_2NC(NH)$-Tyr-D-Arg-Phe-(R)-$NHCH(C_2H_5)COOH$ | 11.17 | 0.39 | 0.59 |
| $H_2NC(NH)$-Tyr-D-Arg-Phe-D-Ala-OH | 9.94 | 0.40 | 0.65 |
| $H_2NC(NH)$-Tyr-Arg-Phe-$N(CH_3)(CH_2)_2COOH$ | 11.65 | 0.33 | 0.54 |
| $H_2NC(NH)$-Tyr-D-Arg-Phe-$NHCH_2CH_2SO_3H$ | 7.67 | 0.38 | 0.68 |
| 1-[$H_2NC(NH)$-Tyr-D-Arg-Phe]-piperidine | 14.87 | 0.66 | 0.80 |
| 1-[$H_2NC(NH)$-Tyr-D-Arg-Phe]-piperazine | 7.55 | 0.28 | 0.60 |
| $H_2NC(NH)$-Tyr-D-Arg-Phe-$N(CH_2C_6H_5)(CH_2)_2COOH$ | 14.87 | 0.51 | 0.70 |
| $H_2NC(NH)$-Tyr-D-Arg-Phe-(R,S)-$NHCH_2CH(CH_3)COOH$ | 9.96 | 0.41 | 0.62 |
| $H_2NC(NH)$-Tyr-D-Arg-Phe-$N(CH_2CH_3)(CH_2)_2COOMe$ | 12.74 | 0.47 | 0.74 |
| $H_2NC(NH)$-Tyr-D-Arg-Phe-Phe-OH | 13.86 | 0.41 | 0.62 |

Table 2

| Peptide derivatives of the present invention |
|---|
| Et-HNC(NH)-Tyr-(D)Arg-Phe-Me β Ala-OH |
| Et-HNC(NH)-Tyr-(D)Arg-Phe-β Ala-OH |
| n-Pr-HNC(NH)-Tyr-(D)Arg-Phe-β Ala-OH |
| Ph-HNC(NH)-Tyr-(D)Arg-Phe-β Ala-OH |
| Ac-HNC(NH)-Tyr-(D)Arg-Phe-β Ala-OH |

Table 2 (continued)

| Peptide derivatives of the present invention |
| --- |
| $H_2NC(NH)$-Tyr-(D)Arg-Phe-NH(n-Pr) |
| $H_2NC(NH)$-Tyr-(D)Arg-Phe-$NH(CH_2)_2OH$ |
| $H_2NC(NH)$-Tyr-(D)Arg-Phe-$NHCH(CH_3)CH_2OH$[D] |
| $H_2NC(NH)$-Tyr-(D)Arg-Phe-$NH(CH_2)_3OH$ |
| $H_2NC(NH)$-Tyr-(D)Arg-Phe-$NH(CH_2)_4OH$ |
| $H_2NC(NH)$-Tyr-(D)Arg-Phe-Gly-OMe |
| $H_2NC(NH)$-Tyr-(D)Arg-Phe-Gly-NHMe |
| $H_2NC(NH)$-Tyr-(D)Arg-Phe-MeGly-OH |
| $H_2NC(NH)$-Tyr-(D)Arg-Phe-Ala-OMe |
| $H_2NC(NH)$-Tyr-(D)Arg-Phe-Ala-$NH_2$ |
| $H_2NC(NH)$-Tyr-(D)Arg-Phe-Ala-NHMe |
| $H_2NC(NH)$-Tyr-(D)Arg-Phe-(D)Ala-NHMe |
| $H_2NC(NH)$-Tyr-(D)Arg-Phe-(D)MeAla-OH |
| $H_2NC(NH)$-Tyr-(D)Arg-Phe-β Ala-OH |
| $H_2NC(NH)$-Tyr-(D)Arg-Phe-Me β Ala-OH |
| $H_2NC(NH)$-Tyr-(D)Arg-Phe-Me β Ala-$NH_2$ |
| $H_2NC(NH)$-Tyr-(D)Arg-Phe-Me β Ala-NHMe |
| $H_2NC(NH)$-Tyr-(D)Arg-Phe-Me β Ala-NHEt |
| $H_2NC(NH)$-Tyr-(D)Arg-Phe-Me β Ala-NH(n-Pr) |

Table 2 (continued)

| Peptide derivatives of the present invention |
|---|
| $H_2NC(NH)$-Tyr-(D)Arg-Phe-Me β Ala-$N(Et)_2$ |
| $H_2NC(NH)$-Tyr-(D)Arg-Phe-Et β Ala-OMe |
| $H_2NC(NH)$-Tyr-(D)Arg-Phe-(D)Arg-$NH_2$ |
| $H_2NC(NH)$-Tyr-(D)Arg-Phe-Asu-OH |
| $H_2NC(NH)$-Tyr-(D)Arg-Phe-Sar-OH |
| $H_2NC(NH)$-Tyr-(D)Arg-Phe-Pro-OH |
| $H_2NC(NH)$-Tyr-(D)Arg-Phe-Pro-OMe |

Experiments

The analgesic activities of the peptide derivatives of the present invention were evaluated by pressure stimulation method. Mice were subjected to pressure stimulation at the bases of their tails at the rate of 10 mmHg/second. Pressure values where mice showed behaviors such as writhing and biting at the stimulated site were measured, and were used as pain reaction thresholds. For the experiments, mice that normally responded to a pressure of 40-50 mmHg were used. The maximum stimulating pressure was 100 mmHg. Analgesic activity was calculated as percent of maximum possible effect (% of MPE) according to the equation:

$$\%\text{ of MPE} = \frac{Pt - Po}{Pc - Po} \times 100$$

wherein Po is the pain reaction threshold before the administration of a drug; Pt is the pain reaction threshold "t" minutes after the administration of the drug; and Pc is the maximum stimulating pressure. $ED_{50}$ values were calculated based on dose response curves as doses that induced 50 percent of % of MPE and analgesic activities of the drugs were compared. The results obtained from subcutaneous administration (skin in the backs) and oral administration are shown in Table 3.

Table 3

| Peptide derivative of the present invention | $ED_{50}$(mg/kg) | |
|---|---|---|
| | s.c. | p.o. |
| Control (morphine) | 4.6 | 29.6 |
| $H_2NC(NH)$-Tyr-D-Arg-Phe-$NHCH_2CH_2COOH$ | 0.14 | 5.8 |
| $H_2NC(NH)$-Tyr-D-Arg-Phe-$NHCH_2CH_2CONH_2$ | 0.13 | 10.7 |
| $H_2NC(NH)$-Tyr-D-Arg-Phe-$N(CH_3)CH_2CH_2COOH$ | 0.10 | 6.6 |
| $H_2NC(NH)$-Tyr-D-Arg-Phe-$N(CH_2CH_3)CH_2CH_2COOH$ | 0.15 | 16.0 |
| $H_2NC(NH)$-Tyr-(D)Arg-Phe-$N(Me)_2$ | 1.22 | *N.T. |
| $H_2NC(NH)$-Tyr-(D)Arg-Phe-$N(CH_3)CH_2CONH_2$ | 0.27 | N.T. |
| $H_2NC(NH)$-Tyr-D-Arg-Phe-$N(CH_3)CH_2COOH$ | 0.39 | 17.8 |
| $H_2NC(NH)$-Tyr-D-Arg-Phe-$NHCH_2CH_2CH_2OH$ | 0.88 | N.T. |
| $H_2NC(NH)$-Tyr-D-Arg-Phe-$N(CH_3)CH_2CH_2CONH$ | 0.31 | 24.6 |
| $H_2NC(NH)$-Tyr-D-Arg-Phe-$N(CH_3)CH_2CH_2COOCH_3$ | 0.12 | 8.9 |
| 1-[$H_2NC(NH)$-Tyr-D-Arg-Phe]-piperidine-3-COOH | 0.25 | N.T. |

* N.T.: not tested

Industrial Applicability

The peptide derivatives of the present invention are useful since they can be used for the treatment of cancerous pain.

## Claims

1. A compound represented by the following formula:

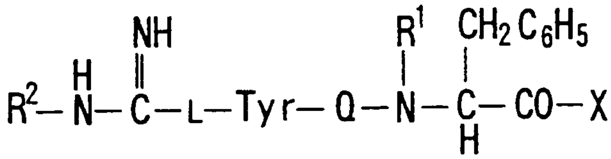

wherein

$R^1$ represents hydrogen atom or a $C_{1-6}$ alkyl group;
$R^2$ represents hydrogen atom, a $C_{1-6}$ alkyl group, an aryl group, a $C_{1-6}$ alkanoyl group, or an arylcarbonyl group;
Q represents D-Arg or L-Arg;
X represents $-OR^3$ wherein $R^3$ represents hydrogen atom or a $C_{1-6}$ alkyl group,
$-NR^4R^5$ wherein $R^4$ represents hydrogen atom or a $C_{1-6}$ alkyl group, and $R^5$ represents a $C_{1-6}$ hydroxyalkyl or a sulfonic acid-substituted $C_{1-6}$ alkyl group, or $R^4$ and $R^5$ may combine together with the nitrogen atom to which $R^4$ and $R^5$ bind to represent a 5 or 6-membered nitrogen containing saturated heterocyclic group,
$-NR^6-CR^7R^8R^9$ wherein $R^6$ represents hydrogen atom, a $C_{1-6}$ alkyl group, or an aryl $C_{1-6}$ alkyl group, $R^7$ represents hydrogen atom, carboxyl group, a $C_{1-6}$ alkoxy carbonyl group, a substituted or non-substituted carbamoyl group, a carboxy-$C_{1-6}$ alkyl group, a substituted or non-substituted carbamoyl $C_{1-6}$ alkyl group, or a $C_{1-6}$ alkoxycarbonyl-$C_{1-6}$ alkyl group, and $R^8$ represents hydrogen atom, a $C_{1-6}$ alkyl group, an amino-$C_{1-6}$ alkyl

group, an amidino-$C_{1-6}$ alkyl group, a guanidino-$C_{1-6}$ alkyl group, a hydroxy $C_{1-6}$ alkyl group, a carboxy-$C_{1-6}$ alkyl group, or a substituted or non-substituted carbamoyl $C_{1-6}$ alkyl group, or $R^6$ and $R^8$ may combine together with the nitrogen atom to which $R^6$ binds to form a 5 or 6-membered nitrogen containing saturated heterocyclic group having a carboxyl group on its ring, and $R^9$ represents hydrogen atom and a $C_{1-6}$ alkyl group.

**2.** The compound or a salt thereof according to claim 1 wherein $R^1$ is hydrogen atom.

**3.** The compound or a salt thereof according to claim 1 or claim 2 wherein $R^6$ is methyl or ethyl group.

**4.** The compound or a salt thereof according to any one of claims 1 to 3 wherein $R^7$ is carboxyl group or carbamoyl group.

**5.** The compound or a salt thereof according to any one of claims 1 to 4 wherein $R^7$ is carboxymethyl group or carbamoylmethyl group.

**6.** The compound or a salt thereof according to any one of claims 1 to 5 wherein Q is D-Arg.

**7.** The compound or a salt thereof according to claim 6 wherein $R^1$ is hydrogen atom, $R^6$ is hydrogen atom, $R^7$ is carboxymethyl group, and $R^8$ is hydrogen atom.

**8.** The compound or a salt thereof according to claim 6 wherein $R^1$ is hydrogen atom, $R^6$ is hydrogen atom, $R^7$ is carbamoylmethyl group, and $R^8$ is hydrogen atom.

**9.** The compound or a salt thereof according to claim 6 wherein $R^1$ is hydrogen atom, $R^6$ is methyl group, $R^7$ is carboxymethyl group, and $R^8$ is hydrogen atom.

**10.** The compound or a salt thereof according to claim 6 wherein $R^1$ is hydrogen atom, $R^6$ is ethyl group, $R^7$ is carboxymethyl group, and $R^8$ is hydrogen atom.

**11.** An oligomer or cyclic compound of the compound according to claim 1 or salts thereof.

**12.** The salt of the compound according to claims 1 to 11 wherein the salt is a hydrochloride or an acetate.

**Patentansprüche**

**1.** Eine Verbindung repräsentiert durch die folgende Formel:

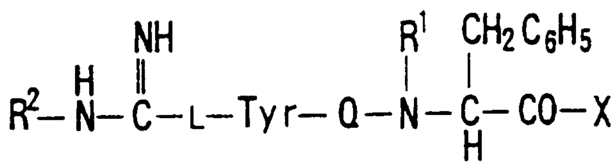

wobei $R^1$ ein Wasserstoffatom oder eine $C_{1-6}$-Alkylgruppe repräsentiert;
$R^2$ ein Wasserstoffatom, eine $C_{1-6}$-Alkylgruppe, eine Arylgruppe, eine $C_{1-6}$-Alkanoylgruppe oder eine Arylcarbonylgruppe repräsentiert;
Q repräsentiert D-Arg oder L-Arg;
X repräsentiert $-OR^3$, wobei $R^3$ ein Wasserstoffatom oder eine $C_{1-6}$-Alkylgruppe repräsentiert,
$-NR^4R^5$, wobei $R^4$ ein Wasserstoffatom oder eine $C_{1-6}$-Alkylgruppe repräsentiert und $R^5$ eine $C_{1-6}$-Hydroxyalkyl- oder eine Sulfonsäuresubstituierte $C_{1-6}$-Alkylgruppe repräsentiert oder $R^4$ und $R^5$ zusammen mit dem Stickstoffatom, an das $R^4$ und $R^5$ gebunden sind, kombiniert werden, um eine 5- oder 6-gliedrige Stickstoff-haltige gesättigte heterocyclische Gruppe, zu repräsentieren,
$-NR^6-CR^7R^8R^9$, wobei $R^6$ ein Wasserstoffatom, eine $C_{1-6}$-Alkylgruppe oder eine Aryl-$C_{1-6}$-Alkylgruppe reprä-

sentiert, $R^7$ ein Wasserstoffatom, eine Carboxylgruppe, eine $C_{1-6}$-Alkoxycarbonylgruppe, eine substituierte oder nicht-substituierte Carbamoylgruppe, eine Carboxy-$C_{1-6}$-Alkylgruppe, eine substituierte oder nicht-substituierte Carbamoyl-$C_{1-6}$-Alkylgruppe oder eine $C_{1-6}$-Alkoxycarbonyl-$C_{1-6}$-Alkylgruppe repräsentiert und $R^8$ ein Wasserstoffatom, eine $C_{1-6}$-Alkylgruppe, eine Amino-$C_{1-6}$-Alkylgruppe, eine Amidino-$C_{1-6}$-Alkylgruppe, eine Guanidino-$C_{1-6}$-Alkylgruppe, eine Hydroxy-$C_{1-6}$-Alkylgruppe, eine Carboxy-$C_{1-6}$-Alkylgruppe oder eine substituierte oder nicht-substituierte Carbamoyl-$C_{1-6}$-Alkylgruppe oder $R^6$ und $R^8$ zusammen mit dem Stickstoffatom, an das $R^6$ gebunden ist, kombiniert werden, um eine 5- oder 6-gliedrige Stickstoff-haltige gesättigte heterocyclische Gruppe zu bilden, die eine Carboxylgruppe in ihrem Ring enthält und $R^9$ ein Wasserstoffatom und eine $C_{1-6}$-Alkylgruppe repräsentiert.

**2.** Eine Verbindung oder deren Salz gemäß Anspruch 1, wobei $R^1$ ein Wasserstoffatom ist.

**3.** Eine Verbindung oder deren Salz gemäß Anspruch 1 oder 2, wobei $R^6$ eine Methyl- oder Ethylgruppe ist.

**4.** Eine Verbindung oder deren Salz gemäß einem der Ansprüche 1 bis 3, wobei $R^7$ eine Carboxyl- oder Carbamoylgruppe ist.

**5.** Eine Verbindung oder deren Salz gemäß einem der Ansprüche 1 bis 4, wobei $R^7$ eine Carboxymethyl- oder Carbamoylmethylgruppe ist.

**6.** Eine Verbindung oder deren Salz gemäß einem der Ansprüche 1 bis 5, wobei Q D-Arg ist.

**7.** Eine Verbindung oder deren Salz gemäß Anspruch 6, wobei $R^1$ ein Wasserstoffatom ist, $R^6$ ein Wasserstoffatom ist, $R^7$ eine Carboxymethylgruppe ist und $R^8$ ein Wasserstoffatom ist.

**8.** Eine Verbindung oder deren Salz gemäß Anspruch 6, wobei $R^1$ ein Wasserstoffatom ist, $R^6$ ein Wasserstoffatom ist, $R^7$ eine Carbamoylmethylgruppe ist und $R^8$ ein Wasserstoffatom ist.

**9.** Eine Verbindung oder deren Salz gemäß Anspruch 6, wobei $R^1$ ein Wasserstoffatom ist, $R^6$ eine Methylgruppe ist, $R^7$ eine Carboxylmethylgruppe ist und $R^8$ ein Wasserstoffatom ist.

**10.** Eine Verbindung oder deren Salz gemäß Anspruch 6, wobei $R^1$ ein Wasserstoffatom ist, $R^6$ eine Ethylgruppe ist, $R^7$ eine Carboxylmethylgruppe ist und $R^8$ ein Wasserstoffatom ist.

**11.** Ein Oligomer oder eine cyclische Verbindung der Verbindung gemäß Anspruch 1 oder Salze davon.

**12.** Ein Salz der Verbindung gemäß den Ansprüchen 1 bis 11, wobei das Salz ein Hydrochlorid oder ein Acetat ist.

**Revendications**

**1.** Une combinaison représentée par la formule suivante:

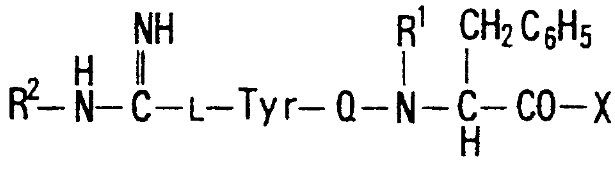

où $R^1$ représente un atome d'hydrogène ou un groupe alkyle $C_{1-6}$;
$R^2$ représente un atome d'hydrogène, un groupe alkyle $C_{1-6}$, un groupe aryle, un groupe alcanoyle $C_{1-6}$ ou un groupe arylecarbonyle;
Q représente D-Arg ou L-Arg;
X représente $-OR^3$, où $R^3$ représente un atome d hydrogène ou un groupe alkyle $C_{1-6}$,

-$NR^4R^5$, où $R^4$ représente un atome d'hydrogène ou un groupe alkyle $C_{1-6}$ et $R^5$ représente un groupe hydroxyalkyle $C_{1-6}$ ou un groupe alkyle $C_{1-6}$ substitué avec de l'acide sulfonique, ou $R^4$ et $R^5$ sont combinés ensemble avec l'atome d'azote auquel $R^4$ et $R^5$ sont liés, afin de représenter un groupe hétérocyclique à 5 ou 6 membres saturé contenant de l'azote,

-$NR^6$-$CR^7R^8R^9$, où $R^6$ représente un atome d'hydrogène, un groupe alkyle $C_{1-6}$ ou un groupe aryle-$C_{1-6}$-alkyle, $R^7$ représente un atome d'hydrogène, un groupe carboxyle, un groupe alcoxycarbonyle $C_{1-6}$, un groupe carbamoyle substitué ou non substitué, un groupe carboxy-$C_{1-6}$-alkyle, un groupe carbamoyle-$C_{1-6}$-alkyle substitué ou non substitué ou un groupe $C_{1-6}$-alcoxycarbonyle-$C_{1-6}$-alkyle, et $R^8$ représente un atome d'hydrogène, un groupe alkyle $C_{1-6}$, un groupe amino-$C_{1-6}$-alkyle, un groupe amidino-$C_{1-6}$-alkyle, un groupe guanidino-$C_{1-6}$-alkyle, un groupe hydroxy-$C_{1-6}$-alkyle, un groupe carboxy-$C_{1-6}$-alkyle ou un groupe carbamoyle-$C_{1-6}$-alkyle substitué ou non substitué, ou $R^6$ et $R^8$ sont combinés ensemble avec l'atome d'azote auquel $R^6$ est lié, afin de former un groupe hétérocyclique à 5 ou 6 membres saturé contenant de l'azote, qui contient un groupe carboxyle dans son composé cyclique, et $R^9$ représente un atome d'hydrogène et un groupe alkyle $C_{1-6}$.

2. Une combinaison ou son sel selon la revendication 1, où $R^1$ est un atome d'hydrogène.

3. Une combinaison ou son sel selon la revendication 1 ou 2, où $R^6$ est un groupe méthyle ou éthyle.

4. Une combinaison ou son sel selon l'une des revendications 1 à 3, où $R^7$ est un groupe carboxyle ou carbamoyle.

5. Une combinaison ou son sel selon l'une des revendications 1 à 4, où $R^7$ est un groupe carboxyméthyle ou un groupe carbamoyleméthyle.

6. Une combinaison ou son sel selon l'une des revendications 1 à 5, où Q est D-Arg.

7. Une combinaison ou son sel selon la revendication 6, où $R^1$ est un atome d'hydrogène, $R^6$ est un atome d'hydrogène, $R^7$ est un groupe carboxyméthyle et $R^8$ est un atome d'hydrogène.

8. Une combinaison ou son sel selon la revendication 6, où $R^1$ est un atome d'hydrogène, $R^6$ est un atome d'hydrogène, $R^7$ est un groupe carbamoyleméthyle et $R^8$ est un atome d'hydrogène.

9. Une combinaison ou son sel selon la revendication 6, où $R^1$ est un atome d'hydrogène, $R^6$ est un groupe méthyle, $R^7$ est un groupe carboxyleméthyle et $R^8$ est un atome d'hydrogène.

10. Une combinaison ou son sel selon la revendication 6, où $R^1$ est un atome d'hydrogène, $R^6$ est un groupe éthyle, $R^7$ est un groupe carboxyleméthyle et $R^8$ est un atome d'hydrogène.

11. Un oligomère ou une combinaison cyclique de la combinaison selon la revendication 1 ou des sels de cela.

12. Un sel de la combinaison selon les revendications 1 à 11, où le sel est un hydrochlorure ou un acétate.